Europäisches Patentamt

European Patent Office

Office européen des brevets

(11) Veröffentlichungsnummer: **0 261 260**
**A1**

## (12) EUROPÄISCHE PATENTANMELDUNG

(21) Anmeldenummer: 86113059.9

(22) Anmeldetag: 23.09.86

(51) Int. Cl.⁴: **A61B 17/16**

(43) Veröffentlichungstag der Anmeldung:
**30.03.88 Patentblatt 88/13**

(84) Benannte Vertragsstaaten:
**AT BE CH DE FR GB IT LI LU NL SE**

(71) Anmelder: **List, Heinz-Jürgen**
**Lindenstrasse 56**
**D-7990 Friedrichshafen 1(DE)**

(72) Erfinder: **List, Heinz-Jürgen**
**Lindenstrasse 56**
**D-7990 Friedrichshafen 1(DE)**
Erfinder: **Richter, Ulrich**
**Ludolfinger Strasse 14**
**D-8500 Nürnberg 50(DE)**

(74) Vertreter: **Göbel, Matthias, Dipl.-Ing.**
**Pruppacher Hauptstrasse 5-7**
**D-8501 Pyrbaum-Pruppach(DE)**

(54) **Chirurgische Knochenbohrmaschine.**

(57) Bei einer chirurgischen Knochenbohrmaschine mit einem Aufnahmegehäuse für den elektromotorischen Antrieb einer Festhaltevorrichtung, insbesondere für Bohrwerkzeuge und einem am Aufnahmegehäuse angeordneten Griffteil, der eine elektrische Stromquelle aufnimmt, ist zur stabilen Halterung und feinfühligen Führung derselben am Griffteil (5) an dem dem Aufnahmegehäuse (I) abgewandten Ende ein über Seitenflächen des Griffteils (5) ragendes Gehäuse (6) für die Unterbringung der Stromquelle angeordnet und der Griffteil (5) in den Seitenflächen abschnittesweise mit Einziehungen (9, I0, II, I2, I3) versehen, die gemeinsam mit den dem Aufnahmegehäuse (I) zugewandten Randflächen (I5) des Gehäuses (6) Stützflächen für eine Mittelhandfläche und für Finger der Benutzerhand bilden.

Fig.1

EP 0 261 260 A1

## Chirurgische Knochenbohrmaschine

Die Erfindung betrifft eine chirurgische Knochenbohrmaschine mit einem Aufnahmegehäuse für den elektromotorischen Antrieb einer Festhaltevorrichtung, insbesondere für Bohrwerkzeuge und einem am Aufnahmegehäuse angeordneten Griffteil, der eine elektrische Stromquelle aufnimmt.

Bei einer bekannten chirurgischen Bohrmaschine (DE-GM 8 230 734) ist das Aufnahmegehäuse für den Antriebsmotor mit einem prismatischen Handgriff versehen, der der gleichzeitigen Aufnahme elektrischer Speicherzellen dient. Abgesehen davon, daß bei dieser Bohrmaschine eine kopflastige Gewichtsverteilung vorliegt, erlaubt der Handgriff nur eine labile Erfassung der Bohrmaschine. Bei einer weiterhin bekannten Knochenbohrmaschine (DE-OS 33 l7 398) nimmt ein Gehäuseoberteil das Getriebe für eine Antriebsspindel auf, während der Antriebsmotor und die elektrische Stromquelle in einem als Griffteil ausgebildeten prismatischen Gehäuseunterteil angeordnet sind. Obwohl diese Bohrmaschine durch die Unterbringung von Antriebsmotor und Stromquelle im Handgriff bereits eine günstigere Gewichtsverteilung aufzeigt, verhindert die Ausbildung des Griffteils jedoch eine sichere und feinfühlige Halterung und Führung der Bohrmaschine.

Es ist Aufgabe der Erfindung Maßnahmen zu einer stabilen Halterung und feinfühligen Führung einer chirurgischen Knochenbohrmaschine zu - schaffen.

Erfindungsgemäß ist diese Aufgabe dadurch gelöst, daß am Griffteil an dem dem Aufnahmegehäuse abgewandten Ende ein über Seitenflächen des Griffteils ragendes Gehäuse für die Unterbringung der Stromquelle angeordnet ist und daß der Griffteil in den Seitenflächen abschnittsweise Einziehungen aufweist, die gemeinsam mit den dem Aufnahmegehäuse zugewandten Randflächen des Gehäuses Stützflächen für eine Mittelhandfläche und für Finger bilden. Bei der so gebildeten Knochenbohrmaschine erstreckt sich der Schwerpunktbereich an günstiger Stelle und es ist eine kraft-und formschlüssige Erfassung des Griffteils möglich. Die Flächenpressung am Griffteil ist so gering gehalten, wodurch eine feinfühlige Handhabung der Knochenbohrmaschine möglich ist. Zweckmäßig ist der Griffteil in Bohrrichtung auf einer Seitenfläche und quer zur Bohrrichtung auf beiden Seitenflächen durch das die Stromquelle aufnehmende Gehäuse überragt.

Gemäß bevorzugter Ausgestaltung der Knochenbohrmaschine ist vorgesehen, daß in Bohrrichtung in einer Seitenfläche des Griffteils eine bis in die Rückseite des Griffteils sich erstreckende Einziehung für die Aufnahme der Mittelhandfläche und des Daumenballens und in der gegenüberliegenden Seitenfläche eine Einziehung für die Aufnahme des Daumens ausgebildet ist und daß die Vorderseite des Griffteils untereinander je eine Einziehung für die Aufnahme des Zeigefingers und des Mittelfingers sowie eine Einziehung für die gemeinsame Aufnahme von Ringfinger und kleinem Finger der Betätigungshand aufweist. Der Griffteil ist auf diese Weise den anatomischen Gegebenheiten der Hand des Benutzers angepaßt. Die Anpassung läßt eine besonders feinfühlige Einleitung der Betätigungskräfte auf den Griffteil zu und erlaubt exakte Bohrvorgänge. Außerdem erstreckt sich die Schwerpunktachse der Knochenbohrmaschine weitgehend im Bereich der Mittelhandfläche und damit ist die Verlängerung der Bohrachse durch das Ellenbogengelenk geführt. Es entspricht dem Erfindungsgedanken, daß die Anordnung der Einziehungen und der Randflächen des Stromquellengehäuses an Links- oder Rechtshänder angepaßt ist.

In weiterer Ausgestaltung sind das die Stromquelle aufnehmende Gehäuse lösbar fest am Griffteil angeordnet und in der gemeinsamen Trennungsebene von Griffteil und Gehäuse mechanische Verbindungselemente und elektrische Kontaktierungsmittel vorgesehen. Die Trennungsebene kann dabei an beliebiger Stelle im oder zum Griffteil vorgesehen sein. Bevorzugt ist der Griffteil durch zwei axiale Griffteilteile gebildet, von denen einer mit dem Aufnahmegehäuse und der andere mit dem Gehäuse der Stromquelle in fester Verbindung steht und die beide in der gemeinsamen Trennungsebene die mechanischen Verbindungselemente und elektrischen Kontaktierungsmittel aufweisen. Es besteht auch die Möglichkeit, den Griffteil insgesamt mit dem die Stromquelle aufnehmenden Gehäuse fest zu verbinden, z.B. einstückig auszubilden und den Griffteil am Aufnahmegehäuse ansteckbar zu machen. Es versteht sich, daß die elektrischen und elektronischen Elemente, insbesondere die der Drehzahl-regelung dienenden Schaltelemente, z.B. Schalttransistoren zu einer Erwärmung führen. Die Erfindung sieht daher Maßnahmen zur Ableitung von Wärme aus dem Aufnahmegehäuse dadurch vor, daß mindestens der die elektrischen Schaltelemente aufnehmende Griffteilteil am freien Ende, d.h. im Bereich der Trennungsebene eine Platte aus einem Werkstoff mit großer Wärmeleitfähigkeit aufweist, die mit den elektronischen Bauelementen zum Zwecke der Wärmeableitung in Flächenkontakt steht.

Zweckmäßig sind das Aufnahmegehäuse und das Gehäuse für die Aufnahme der Stromquelle sowie der Griffteil bzw. die Griffteilteile durch Hälften gebildet, die im Bereich der Trennungsebene vermittels Dichtungselementen und/oder durch Verschweißen miteinander dicht verbunden sind. Die Knochenbohrmaschine bzw. deren Teile sind auf diese Weise gas-und wasserdicht und sicher sterilisierbar. Bevorzugt ist das Aufnahmegehäuse, der Griffteil und das Gehäuse aus einer mechanisch und thermisch hochbelastbaren Kunststoffmasse ausgeformt, wozu sich als Werkstoff insbesondere faserverstärktes Polyester oder ein Epoxydharz als geeignet erwiesen hat. Die Herstellung von Aufnahmegehäuse, Griffteil und Stromquellengehäuse kann im Spritzguß-oder Laminierverfahren erfolgen.

Zur Steuerung bzw. Regelung des elektromotorischen Antriebs sind das Aufnahmegehäuse und der Griffteil bzw. die Griffteilteile mit Betätigungs-und Kontaktierungsmitteln versehen, die dicht durch Wanddurchführungen hindurchgeführt sind.

Anstelle einer getrennten Ausbildung von mechanischen Verbindungs-und Kontaktierungsmitteln ist noch vorgesehen, die mechanischen Verbindungsmittel gleichzeitig als Kontaktierungsmittel verwendbar zu machen. Hierzu können z.B. druckknopfartige Verbindungselemente an einem Griffteilteil angeordnet sein, die bajonettartig in Öffnungen des anderen Griffteilteils eingreifen und jeweils einen, z.B. mittig isoliert geführten Kontakt aufweisen, die an Kontakten des anderen Griffteilteils pressend zur Anlage kommen.

Wie die Erfindung ausführbar ist, zeigt das in der Zeichnung dargestellte Ausführungsbeispiel. Hierin bedeuten:

Fig. I eine Knochenbohrmaschine in Seitenansicht,

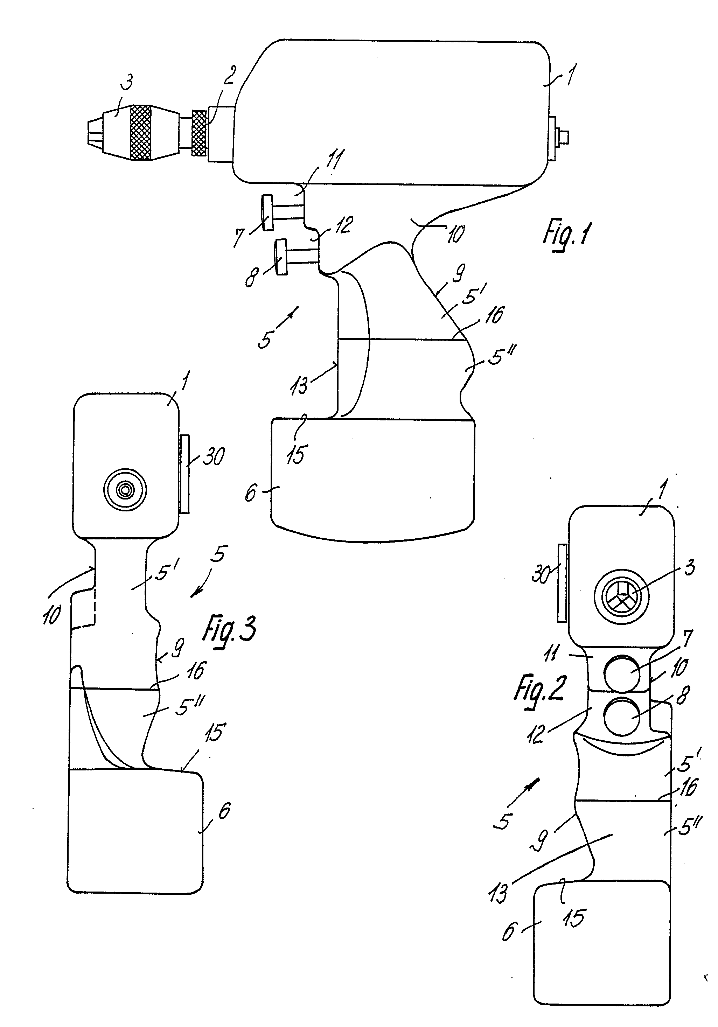

Fig. 2 eine Knochenbohrmaschine in Vorderansicht,

Fig. 3 eine Knochenbohrmaschine in Rückansicht,

Fig. 4 ein Aufnahmegehäuseteilstück einer Knochenbohrmaschine in Unteransicht,

Fig. 5 ein weiteres Teilstück einer Knochenbohrmaschine in Draufsicht,

Fig. 6 ein Teilstück einer Knochenbohrmaschine perspektivisch,

Fig. 7 einen Teilschnitt eines Verbindungselementes vergrößert und

Fig. 8 einen Teilschnitt einer Trennungsebene von zwei Griffteilteilen.

In den Fig. ist mit I das Aufnahmegehäuse einer Knochenbohrmaschine für einen nicht näher dargestellten elektromotorischen Antrieb bezeichnet, dessen Abtriebswelle 2 ein Spannfutter 3 für die Fixierung von, z.B. Bohrwerkzeugen, Bohrdrähten, Lichtleitfasernstrang von Endoskopen trägt. Unterseitig ist am Aufnahmegehäuse I ein Griffteil 5 angeordnet, an dessen freiem Ende sich ein Gehäuse 6 für die Aufnahme einer Stromquelle, z.B. Akku, für den elektromotorischen Antrieb anschließt. Mittels Drucktasten 7 und 8 ist der elektromotorische Antrieb auf Links-bzw. Rechtslauf schaltbar und durch einen Schalthebel 30 ein Getriebe beeinflußbar. Der Griffteil 5 weist in Bohrrichtung in einer Seitenfläche eine sich bis in die Rückseite desselben erstreckende Einziehung 9 auf, die der Aufnahme der Mittelhandfläche und des Daumenballens einer Hand des Benutzers (nicht dargestellt) dient. Ferner ist in der gegenüberliegenden Seitenfläche des Griffteils 5 eine Einziehung I0 angeordnet, die vom Daumen der Benutzerhand durchgreifbar ist, während in der Vorderseite des Griffteils 5 untereinander Einziehungen II und I2 für die Aufnahme des Zeigefingers und des Mittelfingers sowie der Drucktasten 7 und 8 und eine Einziehung I3 für die gemeinsame Aufnahme von Ringfinger und kleinem Finger der Benutzerhand ausgebildet sind. Das Gehäuse 6 ist ausserdem auf den die Einziehungen 9 bis I3 aufweisenden Seiten über den Griffteil 5 seitlich hinausgeführt und bildet mit seinen dem Griffteil zugewandten Randflächen I5 Stützflächen für die Handkante. Mittels der Einziehungen 9 bis I3 und der Randflächen I5 ist der Griffteil 5 form-und kraftschlüssig erfaßbar und dadurch mit geringer Flächenpressung eine feinfühlige Handhabung der Knochenbohrmaschine zu erreichen. Es ist eine ergonomisch anatomisch günstige Griffform geschaffen. Die Anordnung des Gehäuses 6 an dem dem Aufnahmegehäuse I abgewandten Ende des Griffteils 5 bringt dabei den Vorteil, daß der Schwerpunkt der Knochenbohrmaschine in einem günstigen Bereich gelegt ist und die von der Benutzerhand aufgebrachten Kräfte mindestens annähernd durch die Schwerpunktachse verlaufen.

Beim Ausführungsbeispiel ist der Griffteil 5 durch zwei axiale Griffteilteile 5', 5" gebildet, die in der ge meinsamen Trennungsebene I6 durch mechanisch starre Verbindungselemente I7, z.B. Zapfen am Griffteilteil 5' und Nuten I8 im Griffteilteil 5" in der Art einer Bajonettverbindung aneinander festlegbar sind. Weiter weist der Griffteilteil 5' einen Zentrierzapfen I9 auf, der zur Ausrichtung beider Griffteilteile 5' und 5" beim Verbindungsvorgang in eine Bohrung 20 des Griffteilteils 5" eintaucht. Darüber hinaus sind die Griffteilteile 5', 5" mit Kontaktgliedern 2I und 22 ausgerüstet, die beim Verbinden beider Griffteilteile 5', 5" miteinander kontaktieren und die Stromquelle an die elektrische Einrichtung im Griffteilteil 5' anlegen.

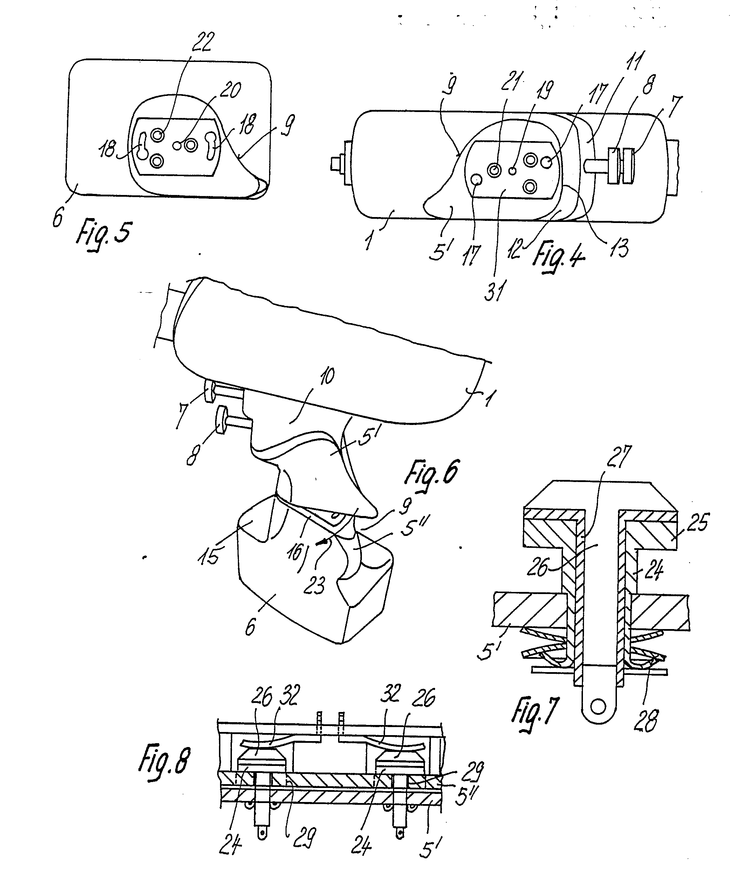

Zum Verbinden der Griffteilteile 5', 5" sind diese zunächst (Fig. 6) verdreht einander gegenübergestellt. Hierbei kann der Zentrierzapfen 19 in die Bohrung 20 eintauchen. Durch einen Verdrehvorgang in Richtung des Pfeiles 23 rasten die Zapfen 17 in die Nuten 18 ein, während die Kontaktglieder 21, 22 miteinander zur Anlage kommen.

In Fig. 7 und 8 ist ein Verbindungselement 24 für die Griffteilteile 5', 5" gezeigt. Das Verbindungselement 24 ist als Hohlzapfen mit einer Verbreiterung 25 ausgeführt, der mittig einen Kontaktstift 26 trägt. Der Kontaktstift 26 ist durch eine Isolierschicht 27 gegenüber dem Hohlzapfen getrennt. Der Hohlzapfen 24 ist mittels Tellerfedern 28 am Griffteilteil 5' abgestützt. Beim Einbringen des Hohlzapfens 24 in eine Schlitzung 29 des Griffteilteils 5" (Fig. 8) wirkt der Hohlzapfen 24 als mechanisches Verbindungselement und als elektrisches Kontaktglied. Zur Kontaktierung kommt der Kontaktstift 26 mit Kontaktkörpern 32 des Griffteilteils 5" zur Anlage. Mit 31 ist eine Platte aus einem Werkstoff mit großer Wärmeleitfähigkeit, z.B. einem metallischen Werkstoff bezeichnet, an die zur Wärmeableitung ein im Griffteilteil 5' untergebrachter Leistungstransistor der elektromotorischen Regelschaltung für die Antriebseinrichtung Flächenkontakt hält. Die Platte 31 kann die Zapfen 17 bzw. 24 tragen.

## Ansprüche

1. Chirurgische Knochenbohrmaschine mit einem Aufnahmegehäuse für den elektromotorischen Antrieb einer Festhaltevorrichtung, insbesondere für Bohrwerkzeuge und einem am Aufnahmegehäuse angeordneten Griffteil, der eine elektrische Stromquelle aufnimmt, dadurch gekennzeichnet, daß am Griffteil (5) an dem dem Aufnahmegehäuse (1) abgewandten Ende ein über Seitenflächen des Griffteils (5) ragendes Gehäuse (6) für die Unterbringung der Stromquelle angeordnet ist und daß der Griffteil (5) in den Seitenflächen abschnittsweise Einziehungen (9, 10, 11, 12, 13) aufweist, die gemeinsam mit den dem Aufnahmegehäuse (1) zugewandten Randflächen (15) des Gehäuses (6) Stützflächen für eine Mittelhandfläche und für Finger der Benutzerhand bilden.

2. Chirurgische Knochenbohrmaschine nach Anspruch 1, dadurch gekennzeichnet, daß der Griffteil (5) in Bohrrichtung auf einer Seitenfläche und quer zur Bohrrichtung auf beiden Seitenflächen durch das die Stromquelle aufnehmende Gehäuse (6) überragt ist.

3. Chirurgische Knochenbohrmaschine nach Anspruch 1, dadurch gekennzeichnet, daß in Bohrrichtung in einer Seitenfläche des Griffteils (5) eine sich bis in die Rückseite erstreckende Einziehung (9) für die Aufnahme der Mittelhandfläche und des Daumenballens und in der gegenüberliegenden Seitenfläche eine Einziehung (10) für die Aufnahme des Daumens ausgebildet ist und daß die Vorderseite des Griffteils (5) untereinander Einziehungen (11, 12) für die Aufnahme des Zeigefingers und des Mittelfingers sowie eine Einziehung (13) für die gemeinsame Aufnahme von Ringfinger und kleinem Finger aufweist.

4. Chirurgische Knochenbohrmaschine nach Anspruch 1, dadurch gekennzeichnet, daß das die Stromquelle aufnehmende Gehäuse (6) lösbar fest am Griffteil (5) angeordnet ist und daß in der gemeinsamen Trennungsebene (16) von Griffteil (5) und Gehäuse (6) mechanische Verbindungselemente (17, 18) und elektrische Kontaktierungsmittel (21, 22) ausgebildet sind.

5. Chirurgische Knochenbohrmaschine nach Anspruch 1 und 4, dadurch gekennzeichnet, daß der Griffteil (5) durch zwei axiale Griffteilteile (5', 5") gebildet ist, von denen einer mit dem Aufnahmegehäuse (1) und der andere mit dem Gehäuse (6) fest verbunden ist und daß die beiden Griffteilteile (5', 5") in der gemeinsamen Trennungsebene (16) mechanische Verbindungselemente (17, 18) und elektrische Kontaktierungsmittel (21, 22) aufweisen.

6. Chirurgische Knochenbohrmaschine nach Anspruch 4 und 5, dadurch gekennzeichnet, daß der mit dem Aufnahmegehäuse (1) verbundene Griffteilteil (5') am freien Ende eine Platte (31) aus einem Werkstoff mit großer Wärmeleitfähigkeit aufweist, an die im Griffteilteil (5') untergebrachte elektronische Bauelemente anliegen.

7. Chirurgische Knochenbohrmaschine nach Anspruch 1 und 5, dadurch gekennzeichnet, daß das Aufnahmegehäuse (1), der Griffteil (5) und das Gehäuse (6) bzw. die Gehäuse (1, 6) und die Griffteilteile (5', 5") durch Hälften gebildet sind, die im Bereich der Trennungsebenen mittels Dichtungselementen und/oder Verschweißen dicht verbunden sind.

8. Chirurgische Knochenbohrmaschine nach Anspruch 1, dadurch gekennzeichnet, daß das Aufnahmegehäuse (1), der Griffteil (5) und das Gehäuse (6) aus mechanisch und thermisch hochbelastbaren Kunststoffmassen ausgeformt ist.

9. Chirurgische Knochenbohrmaschine nach Anspruch 8, gekennzeichnet durch die Anordnung von faserverstärkten Kunststoffmassen für die Ausbildung von Aufnahmegehäuse (1), Griffteil (5) und Gehäuse (6).

10. Chirurgische Knochenbohrmaschine nach Anspruch 7, dadurch gekennzeichnet, daß das Aufnahmegehäuse (1) und die Griffteilteile (5', 5") zur Steuerung und Regelung der elektromotorischen Antriebseinrichtung Betätigungsmittel (7, 8, 30) und

Kontaktierungsmittel (21, 22) aufweisen und daß die Wanddurchführungen für diese dicht ausgebildet sind.

11. Chirurgische Knochenbohrmaschine nach Anspruch 4, 5 und 10, dadurch gekennzeichnet, daß als mechanische Verbindungselemente am Griffteil-teil (5') Hohlzapfen (24) mit einer Verbreiterung (25) angeordnet sind, die als elektrisches Kontaktierungsmittel isoliert einen Kontaktstift (26) aufnehmen und daß die Hohlzapfen (24) in Schlitzungen (29) des Griffteilteils (5") einschwenkbar sind und eingeschwenkt die Kontaktstifte (26) an Kontaktkörper (32) anlegen.

0 261 260

Fig. 1

Fig. 3

Fig. 2

Fig.5

Fig.4

Fig.6

Fig.7

Fig.8

Europäisches
Patentamt

**EUROPÄISCHER RECHERCHENBERICHT**

Nummer der Anmeldung

EP 86 11 3059

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-3 734 207 (H.FISHBEIN)<br><br>* Das ganze Dokument *<br><br>--- | 1,3-5, 8-11 | A 61 B 17/16 |
| Y | US-A-3 128 571 (S.J.HERRETT)<br><br>* Abbildungen 1-3 *<br><br>--- | 1,3-5, 8-11 | |
| Y | METALWORKING PRODUCTION, Band 108, Nr. 1, 1. Januar 1964, Seiten 53-55, London, GB; "Total insulation makes portable-tool earthing obsolete"<br>* Seite 54, Spalte II, Zeilen 10-19 *<br><br>----- | 8-11 | |

| RECHERCHIERTE SACHGEBIETE (Int. Cl.4) |
|---|
| A 61 B<br>B 23 B<br>F 41 C |

Der vorliegende Recherchenbericht wurde für alle Patentansprüche erstellt.

| Recherchenort | Abschlußdatum der Recherche | Prüfer |
|---|---|---|
| DEN HAAG | 07-05-1987 | ARGENTINI A. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
 
& : Mitglied der gleichen Patentfamilie, überein-stimmendes Dokument

EPA Form 1503 03 82